# EUROPÄISCHE PATENTSCHRIFT

(11) **EP 3 313 826 B1**
(45) Veröffentlichungstag und Bekanntmachung des Hinweises auf die Patenterteilung: **08.01.2020**
(21) Anmeldenummer: 16730415.3
(22) Anmeldetag: 21.06.2016
(51) Int. Cl.: C07D 251/24, C07C 255/57, C07C 253/30, G11B 7/24044, G03C 7/02, G03F 7/00, G11B 7/245

(54) **SUBSTITUIERTE TRIAZINE UND EIN VERFAHREN ZU IHRER HERSTELLUNG**
SUBSTITUTED TRIAZINES AND A METHOD FOR THEIR PREPARATION
TRIAZINES SUBSTITUEES ET UN PROCEDE DE LEUR PREPARATION

(30) Priorität: 23.06.2015 EP 15173235
(43) Veröffentlichungstag der Anmeldung: 02.05.2018
(73) Patentinhaber: Covestro Deutschland AG, 51373 Leverkusen (DE)
(72) Erfinder: RÖLLE, Thomas, 51381 Leverkusen (DE); KAWAMURA, Koichi, 51519 Odenthal (DE); CHIOVETTA, Giuseppe, 50354 Hürth Leverkusen (DE)
(74) Vertreter: Levpat
(86) Internationale Anmeldenummer: PCT/EP2016/064305
(87) Internationale Veröffentlichungsnummer: WO 2016/207158

(56) Entgegenhaltungen:
- EP-A1- 0 332 042
- EP-A1- 2 450 387
- EP-A1- 2 774 921
- US-A1- 2005 026 081
- YOSHITO TOBE ET AL: "Synthesis and Association Behavior of [4.4.4.4.4.4]Metacyclophanedodecayne Derivatives with Interior Binding Groups", ANGEW. CHEM. INT. ED., Bd. 37, Nr. 9, 18. Mai 1998 (1998-05-18), Seiten 1285-1287, XP055205941,
- LOUPY A ET AL: "Synthesis of Long Chain Aromatic Esters in a Solvent-Free Procedure Under Microwaves", TETRAHEDRON, ELSEVIER SCIENCE PUBLISHERS, AMSTERDAM, NL, Bd. 52, Nr. 19, 6. Mai 1996 (1996-05-06), Seiten 6705-6712, XP004189702, ISSN: 0040-4020, DOI: 10.1016/0040-4020(96)00321-3
- ANTHONY G. M. BARRETT ET AL: "Nucleophilic Substitution Reactions of (Alkoxymethylene)dimethylammonium Chloride", THE JOURNAL OF ORGANIC CHEMISTRY, Bd. 63, Nr. 18, 1. September 1998 (1998-09-01), Seiten 6273-6280, XP055205955, ISSN: 0022-3263, DOI: 10.1021/jo980583j
- MAHABALESHWARA SUBRAO ET AL: "Novel biphenyl-substituted 1,2,4-oxadiazole ferroelectric liquid crystals: synthesis and characterization", BEILSTEIN JOURNAL OF ORGANIC CHEMISTRY, Bd. 11, 11. Februar 2015 (2015-02-11), Seiten 233-241, XP055205964, DOI: 10.3762/bjoc.11.26
- DATABASE PubChem Compound [Online] NCBI; 13. Februar 2015 (2015-02-13), XP002743090, Database accession no. CID 89215661
- MARKWARDT F ET AL: "Ueber die antifibrinolytische Wirkung von Estern der 4-Aminomethylbentoesaeure (PAMBA)", DIE PHARMAZIE, GOVI VERLAG PHARMAZEUTISCHER VERLAG GMBH, ESCHBORN, DE, Bd. 21, 1. Januar 1966 (1966-01-01), Seiten 345-348, XP009185680, ISSN: 0031-7144
- REINHOLD DIEING ET AL: "Soluble Substituted [mu]-Oxo(phthalocyaninato)iron(III) Dimers", CHEMISCHE BERICHTE, Bd. 128, Nr. 6, 25. November 1994 (1994-11-25), Seiten 589-598, XP055229347, DE ISSN: 0009-2940, DOI: 10.1002/cber.19951280611
- KRASIK ET AL: "Synthesis of sterically hindered esters via titanium catalyzed transesterification", TETRAHEDRON LETTERS, PERGAMON, GB, Bd. 39, Nr. 24, 11. Juni 1998 (1998-06-11) , Seiten 4223-4226, XP005222467, ISSN: 0040-4039, DOI: 10.1016/S0040-4039(98)00790-4

## Beschreibung

Die vorliegende Erfindung betrifft ein Verfahren zur Herstellung von aromatischen 4,6-Bis-trichlormethyl-s-triazin-2-yl-Verbindungen der Formel (I) sowie ein Verfahren zur Herstellung von para-disubstituierten Benzoesäurenitrilen als Vorstufe zu Verbindungen der Formel (I).

Aus der nicht vor-veröffentlichten europäischen Anmeldung EP13198913.9 sind lichtempfindliche Photopolymere bekannt, die Polyurethanmatrixpolymere, ein Schreibmonomer auf Acrylatbasis sowie Photoinitiatoren enthaltend zwei Coinitiatoren und einen Farbstoff umfassen. Bei der Verwendungen von Photopolymeren spielt die durch die holographische Belichtung erzeugte Brechungsindexmodulation Δn die entscheidende Rolle. Bei der holographischen Belichtung wird das Interferenzfeld aus Signal- und Referenzlichtstrahl (im einfachsten Fall das zweier ebener Wellen) durch die lokale Photopolymerisation von Schreibmonomeren wie z.B. hochbrechenden Acrylate an Orten hoher Intensität im Interferenzfeld in ein Brechungsindexgitter abgebildet. Das Brechungsindexgitter im Photopolymer (das Hologramm) enthält alle Information des Signallichtstrahls. Durch Beleuchtung des Hologramms nur mit dem Referenzlichtstrahl kann dann das Signal wieder rekonstruiert werden. Die Stärke des so rekonstruierten Signals im Verhältnis zur Stärke des eingestrahlten Referenzlichts wird Beugungseffizienz, im Folgenden DE wie Diffraction Efficiency, genannt.

Im einfachsten Fall eines Hologramms, das aus der Überlagerung zweier ebener Wellen entsteht, ergibt sich die DE aus dem Quotienten der Intensität des bei der Rekonstruktion abgebeugten Lichtes und der Summe der Intensitäten aus nicht gebeugten und abgebeugtem Licht. Je höher die DE desto effizienter ist ein Hologramm in Bezug auf die Lichtmenge des Referenzlichtes die notwendig ist, um das Signal mit einer festen Helligkeit sichtbar zu machen.

Um ein möglichst hohes Δn und eine möglichst hohe DE bei Hologrammen realisieren zu können, sollten grundsätzlich die Matrixpolymere und die Schreibmonomere einer Photopolymer-Formulierung so gewählt werden, dass sie sich in ihren Brechungsindizes möglichst stark unterscheiden. Ein Möglichkeit zur Realisierung ist, Matrixpolymere mit einem möglichst niedrigen und Schreibmonomere mit einem möglichst hohen Brechungsindex zu verwenden. Geeignete Matrixpolymere mit niedrigem Brechungsindex sind beispielsweise durch Umsetzung einer Polyol- mit einer Polyisocyanat-Komponente erhältliche Polyurethane.

Neben hohen DE- und Δn- Werten ist es für holographische Medien aus Photopolymer-Formulierungen aber auch von großer Bedeutung, dass die Matrixpolymere im fertigen Medium hoch vernetzt sind. Falls der Vernetzungsgrad zu niedrig ist, weist das Medium keine ausreichende Stabilität auf. Dies kann dazu führen, dass die Qualität von in die Medien eingeschriebenen Hologrammen erheblich vermindert ist. Im schlimmsten Fall können die Hologramme sogar nachträglich zerstört werden.

Weiterhin ist es insbesondere für den großtechnischen Einsatz von holographischen Medien aus Photopolymer-Formulierungen von großer Bedeutung, dass die Lichtempfindlichkeit ausreicht, um bei einer gegebenen Laser-Lichtquelle großflächig und ohne Verlust an Indexmodulation belichten zu können. Hier ist insbesondere die Wahl eines geeigneten Photoinitiators von entscheidender Bedeutung für die Eigenschaften des Photopolymers.

Die holographische Belichtung mit einer kontinuierlichen Laserquelle stößt jedoch bei großflächiger Belichtung an technische Grenzen, da immer eine gewisse Lichtdosis pro Flächeneinheit eingestrahlt werden muss, um eine effiziente Bildung des Hologramms zu gewährleisten und die technisch verfügbare Laserleistung begrenzt ist. Zudem erfordern großflächige Belichtungen bei vergleichsweise geringer Dosis lange Belichtungszeiten, die wiederum sehr hohe Anforderungen an die mechanische Schwingungsdämpfung des Belichtungsaufbaus stellen.

Eine weitere Möglichkeit zur großflächigen Belichtung von Hologrammen besteht in der Verwendung von sehr kurzen Lichtpulsen, wie sie z.B. von gepulsten Lasern oder Dauerstrichlasern in Verbindung mit sehr schnellen Shuttern erzeugt werden. Typische Pulsdauern bei gepulsten Lasern sind 500 ns oder kürzer. Typische Pulsdauern bei Dauerstrichlasern mit sehr schnellen Shuttern sind 100 µs oder kürzer. Dabei kann die gleiche Energiedosis eingestrahlt werden wie mit kontinuierlichen Lasern in Sekunden. Auf diese Art können Hologramme Punkt für Punkt geschrieben werden. Da gepulste Laser oder schnelle optische Shutter technisch verfügbar sind und ein derartiger Belichtungsaufbau sehr geringe Anforderungen mit Hinblick auf die mechanische Schwingungsdämpfung hat, handelt es sich um eine gute technisch Alternative zu den oben beschriebenen Aufbauten mit kontinuierlichen Lasern zur großflächigen Belichtung von Hologrammen.

Die aus der europäischen Anmeldung EP13198913.9 bekannten Photopolymere sind grundsätzlich zum Schreiben von Hologrammen mit gepulsten Lasern verwendbar, allerdings ist für eine breite technische Nutzbarkeit eine weitere Steigerung der Empfindlichkeit des Photopolymers erstrebenswert.

Offenbart ist eine Verbindung der Formel (I), 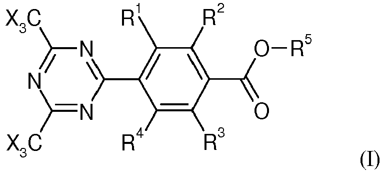 dadurch gekennzeichnet, dass X gleich Halogen und R¹, R², R³ und R⁴ unabhängig voneinander Wasserstoff, Halogen, Alkyl, Alkoxy, Alkenyl, Alkinyl, Alkylthio, Alkylseleno, wobei R¹ und R² und / oder R³ und R⁴ zusammen eine 3 bis 5-gliedrige gesättigte oder ungesättige gegebenenfalls mit bis zu 2 Heteroatomen substituierte Brücke sein können und R⁵ gegebenenfalls beliebig durch Halogen und / oder C₁ bis C₁₀-Alkyl-substituiertes und / oder C₁ bis C₁₀-Alkoxy-substituiertes lineares C₅ bis C₂₀-Alkyl, in dem bis zu 6 Kohlenstoff-Atome durch Sauerstoff ersetzt sein können, wobei zwischen zwei Sauerstoff-Atomen mindestens 2 Kohlenstoff-Atome vorhanden sein müssen und der Rest R⁵ mit mindestens 2 Kohlenstoff-Atomen beginnt, wobei die endständige Methyl-Gruppe des linearen C₅ bis C₂₀-Alkyl-Rests unsubstituiert sein muss, sind.

Medien, die eine Verbindung der Formel (I) enthalten, weisen eine höhere Lichtempfindlichkeit auf und sind damit gut zur Belichtung mit gepulsten Lasern geeignet.

Der Rest X kann für Chlor stehen. Auch offenbart ist, dass R¹, R², R³ und R⁴ für Wasserstoff stehen. R⁵ kann gegebenenfalls beliebig durch Halogen und / oder C₁ bis C₄-Alkyl-substituiertes und / oder C₁ bis C₄-Alkoxy-substituiertes lineares C₆ bis C₂₀-Alkyl sein, in dem bis zu 6 Kohlenstoff-Atome durch Sauerstoff ersetzt sein, wobei zwischen zwei Sauerstoff-Atomen mindestens 2 Kohlenstoff-Atome vorhanden sein müssen und der Rest R⁵ mit mindestens 2 Kohlenstoff-Atomen beginnt, wobei die endständige Methyl-Gruppe des linearen C₆ bis C₂₀-Alkyl-Rests unsubstituiert sein muss.

Ebenfalls offenbart ist, dass bevorzugt ist, wenn bei der Verbindung der Formel (I) der Rest X für Chlor, die Reste R¹, R², R³ und R⁴ für H und der Rest R⁵ für 2-Ethylhexanol steht.

Gemäß EP 0332 042 A2 lassen sich 4,6-Bis-trichlormethyl-s-triazin-2-yl-phenyl-Derivate durch Cyclocotrimerisierung aus aromatischen Nitrilen und Trichloracetonitril herstellen. Dazu werden jeweils die 3- oder 4-Cyano-substituierten Benzoe- oder Zimtsäuremethylester mit Trichloracetonitril umgesetzt und dann der resultierende 4,6-Bis-trichlormethyl-s-triazin-substituierte Benzoe- oder Zimtsäuremethylester aufwendig, teils mehrstufig, umgeestert.

Mahabaleshwara Subrao et al., Beilstein Journal of Organic Chemistry, 11, 2015, Seiten 233-241 offenbart die Synthese und Charakterisierung von neuen Biphenyl-substituierten 1,2,4-oxadizol ferrolectrischen Kristallen.

Markwardt F et al., die Pharmazie 21, 1966, Seiten 345-348 offenbart die antifibrionolytische Wirkung von Estern der 4-Aminomethylbenzoesäure (PAMBA) sowie die Synthese eines Benzonitrilesters.

R. Dieing et al., Chemische Berichte, 128 (6), 1994, Seiten 589-598 offenbart lösliche substituierte µ-Oxo(phthalocyaninato)eisen(III) Dimere Aufgabe war es daher, ein einfaches wirtschaftliches Verfahren zur Herstellung der Verbindungen (I) und deren Vorstufen bereitzustellen.

Diese Aufgabe wurde gelöst durch
ein Verfahren zu Herstellung von para-disubstituierten Benzoesäureester-nitrilen der Formel (IV) 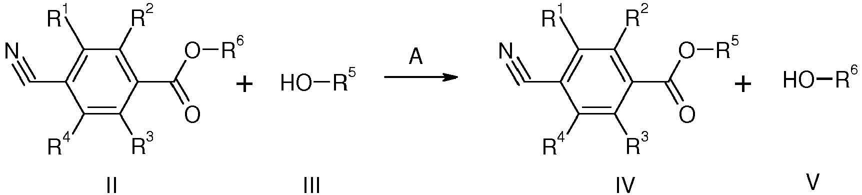 wobei die Reste R¹ bis R⁵ für diejenigen stehen, die unter Formel (I) beschrieben sind, und R⁶ für Methyl oder Ethyl oder isomeres Propyl und A für einen Katalysator A steht, und es sich um ein Ein-Topf-Verfahren handelt, bei dem die Komponente (III) im Überschuss eingesetzt wird, die Komponenten V, III und IV in angegebener Reihenfolge in drei Schritten durch Anpassung des Drucks und der Temperatur destillativ abgetrennt werden und die Differenz der Siedepunkte der Verbindungen (V) und (III) und die der Siedepunkte der Verbindungen (III) und (IV) jeweils mindestens 50 °C beträgt, wobei der Katalysator A ein basischer Katalysator, ein Titan(IV)- oder Zirkonium(IV)-Alkanoat, ein Alkoxy-trialkyl-Zinn oder ein Kupferalkanoat/Triphenylphosphan-Komplex ist.

Dabei wird - mit Bedeutung der Reste R¹ bis R⁴ wie unter Formel (I) beschrieben - ein Ester der Formel (II), gekennzeichnet dadurch, dass R⁶ Methyl oder Ethyl oder die isomeren Propyle sind, in Gegenwart eines Alkohols (III) mit der Bedeutung R⁵ wie oben beschrieben in Anwesenheit eines Katalysators A in einem Ein-Topf-Verfahren umgesetzt, und im ersten Schritt bei einer Innentemperatur von 100 - 150 °C bei einem Druck von 300 - 1000 mbar mit einem Überschuss von 1.1 bis 10 Äquivalenten (III) bis zur vollständigen Umsetzung von (II) erhitzt und (V) destillativ aus dem Reaktionsgemisch entfernt und in einem nächsten Schritt der überschüssige Alkohol (III) bei Kopftemperatur von 50 - 150 °C und bei einem Druck von 1 - 300 mbar destillativ entfernt und einem nächsten Schritt das Produkt (IV) bei Kopftemperatur von 100 - 250 °C und bei einem Druck von 0.01 - 1 mbar destilliert, wobei der Katalysator A im Sumpf verbleibt.

Im Schritt 1 ist eine Innentemperatur von 100 - 150 °C, bevorzugt 110 - 145 °C, besonders bevorzugt 120 - 140 °C bei einem Druck von 300 - 1000 mbar, bevorzugt von 350 - 800 mbar besonders bevorzugt von 400 - 600 mbar mit einem Überschuss von 1.1 bis 10 Äquivalenten (III), bevorzugt von 1.2 bis 5 Äquivalenten, besonders bevorzugt von 1.3 bis 2 Äquivalenten vorgesehen.

Im zweiten Schritt wird eine Kopftemperatur von 50 - 150 °C, bevorzugt 60 - 125 °C, besonders bevorzugt 70 - 100 °C und ein Druck von 1 - 300 mbar, bevorzugt 3 - 100 mbar, besonders bevorzugt 5 - 50 mbar genutzt.

Im dritten Schritt ist eine Kopftemperatur von 100 - 250 °C, bevorzugt von 125 - 240 °C, besonders bevorzugt von 150 - 230 °C und bei ein Druck von 0.01 - 1 mbar, bevorzugt von 0.02 - 0.5 mbar vorgesehen.

Als Alkohol (III) eignen sich alle Verbindungen, die mit mindestens fünf und bis zu 20 aliphatischen gegebenenfalls mit Halogen oder Sauerstoff substituierte Kohlenstoff-Atomen versehen sind, die mindestens eine Methyl-Gruppe tragen. Bevorzugt sind Verbindungen, die mit mindestens sechs und bis zu 15 aliphatischen gegebenenfalls mit Halogen oder Sauerstoff substituierte Kohlenstoff-Atomen versehen sind, die mindestens eine Methyl-Gruppe tragen. Besonderes bevorzugt sind Verbindungen, die mit mindestens sieben und bis zu 10 aliphatischen gegebenenfalls mit Halogen oder Sauerstoff substituierte Kohlenstoff-Atomen versehen sind, die mindestens eine Methyl-Gruppe tragen.

Als Katalysator A eignen sich die grundlegend in Methoden der organischen Chemie (Houben-Weyl), Band E5, 1, III.a) γ, γ₁ (S. 702 ff, Pielartzik, Irmscher-Pielartzik und Eicher) beschriebenen Katalysatoren wie basische Katalysatoren (Alkanoate, Anionenaustauscher), Titan(IV) und Zirconium(IV)-Alkanoate, Alkoxy-trialkyl-Zinn sowie Kupferalkanoat/Triphenylphosphan-Komplexe. Bevorzugt ist, wenn die Katalysatoren in flüssiger Form vorliegen, einen höheren Siedepunkt als das gewünschte Produkt aufweisen und in geringer Konzentration einen raschen und vollständigen Umsatz ermöglichen. Besonders bevorzugt sind daher höhere Titan(IV) und Zirkon(IV)-Alkanoate. Insbesondere bevorzugt sind höhere Titan(IV) und Zirconium(IV)-Alkanoate, bei denen das Alkanoat dem der Verbindung (III) entspricht.

Desweiteren ist bevorzugt, dass der Katalysator A in einer Menge von 0.0001 bis 0.1 Äquivalenten bezüglich der eingesetzten Verbindung (II), besonders bevorzugt von 0.0005 bis 0.01 Äquivalenten und insbesondere bevorzugt 0.001 bis 0.005 Äquivalenten eingesetzt wird.

Weiterhin Gegenstand der Erfindung ist ein Verfahren zur Herstellung der Verbindungen (I), wobei in einem ersten Schritt das Eintopf-Verfahren zu Herstellung von para-disubstituierten Benzoesäurenitrilen der Formel (IV) angewendet wird und in einem zweiten Schritt das resultierende para-disubstituierten Benzoesäurenitril mit Trihalogenoacetonitril umgesetzt wird.

Auch offenbart ist eine Photopolymer-Formulierung enthaltend eine photopolymerisierbare Komponente und mindestens eine Verbindung der Formel (I).

In einer Ausführungsform der Photopolymer-Formulierung enthält diese Matrixpolymere, mindestens ein Schreibmonomer und ein Photoinitiatorsystem enthaltend mindestens eine Verbindung der Formel (I).

Die Matrixpolymere der offenbarten Photopolymer-Formulierung können insbesondere vernetzt und besonders bevorzugt dreidimensional vernetzt sein.

Vorteilhaft ist auch, wenn die Matrixpolymere Polyurethane sind, wobei die die Polyurethane insbesondere durch Umsetzung wenigstens einer Polyisocyanat-Komponente a) mit wenigstens einer Isocyanat-reaktiven-Komponente b) erhältlich sein können.

Die Polyisocyanat-Komponente a) umfasst bevorzugt wenigstens eine organische Verbindung mit wenigstens zwei NCO-Gruppen. Bei diesen organischen Verbindungen kann es sich insbesondere um monomere Di- und Triisocyanate, Polyisocyanate und / oder NCO-funktionelle Prepolymere handeln. Die Polyisocyanat-Komponente a) kann auch Mischungen monomerer Di- und Triisocyanate, Polyisocyanate und / oder NCO-funktioneller Prepolymere enthalten oder daraus bestehen.

Als monomere Di- und Triisocyanate können alle dem Fachmann an sich gut bekannten Verbindungen oder deren Mischungen eingesetzt werden. Diese Verbindungen können aromatische, araliphatische, aliphatische oder cycloaliphatische Strukturen aufweisen. In untergeordneten Mengen können die monomeren Di- und Triisocyanate auch Monoisocyanate, d.h. organische Verbindungen mit einer NCO-Gruppe umfassen.

Beispiele für geeignete monomere Di- und Triisocyanate sind 1,4-Butandiisocyanat, 1,5-Pentandiisocyanat, 1,6-Hexandiisocyanat (Hexamethylendiisocyanat, HDI), 2,2,4-Trimethylhexamethylendiisocyanat und / oder 2,4,4-Trimethylhexamethylendiisocyanat (TMDI), Isophorondiisocyanat (IPDI), 1,8-Diisocyanato-4-(isocyanatomethyl)-octan, Bis-(4,4'-isocyanatocyclohexyl)methan und / oder Bis-(2',4-isocyanatocyclohexyl)methan und / oder deren Mischungen beliebigen Isomerengehalts, 1,4-Cyclohexandiisocyanat, die isomeren Bis-(isocyanatomethyl)cyclohexane, 2,4- und / oder 2,6-Diisocyanato-1-methylcyclohexan (Hexahydro-2,4- und / oder 2,6-toluylendiisocyanat, H₆-TDI), 1,4-Phenylendiisocyanat, 2,4- und / oder 2,6-Toluylendiisocyanat (TDI), 1,5-Naphthylendiisocyanat (NDI), 2,4'- und / oder 4,4'-Diphenylmethandiisocyanat (MDI), 1,3-Bis(isocyanatomethyl)benzol (XDI) und / oder das analoge 1,4-Isomere oder beliebige Mischungen der vorgenannten Verbindungen.

Geeignete Polyisocyanate sind Verbindungen mit Urethan-, Harnstoff-, Carbodiimid-, Acylharnstoff-, Amid-, Isocyanurat-, Allophanat-, Biuret-, Oxadiazintrion-, Uretdion- und/oder Iminooxadiazindionstrukturen, die aus den vorgenannten Di- oder Triisocyanaten erhältlich sind.

Besonders bevorzugt handelt es sich bei den Polyisocyanaten um oligomerisierte aliphatische und / oder cycloaliphatische Di- oder Triisocyanate, wobei insbesondere die oben stehenden aliphatischen und / oder cycloaliphatischen Di- oder Triisocyanate verwendet werden können.

Ganz besonders bevorzugt sind Polyisocyanate mit Isocyanurat-, Uretdion- und / oder Iminooxadiazindion-Strukturen sowie Biurete basierend auf HDI oder deren Mischungen.

Geeignete Prepolymere enthalten Urethan- und / oder Harnstoff-Gruppen sowie gegebenenfalls weitere durch Modifizierung von NCO-Gruppen entstandene Strukturen wie oben genannt. Derartige Prepolymere sind beispielsweise durch Umsetzung der oben genannten monomeren Di- und Triisocyanate und / oder Polyisocyanaten a1) mit isocyanatreaktiven Verbindungen b1) erhältlich.

Als isocyanatreaktive Verbindungen b1) können Alkohole, Amino oder Mercapto-Verbindungen, bevorzugt Alkohole, verwendet werden. Dabei kann es sich insbesondere um Polyole handeln. Ganz besonders bevorzugt können als isocyanatreaktive Verbindung b1) Polyester-, Polyether-, Polycarbonat-, Poly(meth)acrylat- und/oder Polyurethan-Polyole verwendet werden.

Als Polyesterpolyole sind beispielsweise lineare Polyesterdiole oder verzweigte Polyesterpolyole geeignet, die in bekannter Weise durch Umsetzung von aliphatischen, cycloaliphatischen oder aromatischen Di- bzw. Polycarbonsäuren bzw. ihren Anhydriden mit mehrwertigen Alkoholen einer OH-Funktionalität ≥ 2 erhalten werden können. Beispiele für geeignete Di- bzw. Polycarbonsäuren sind mehrwertige Carbonsäuren wie Bernstein-, Adipin-, Kork-, Sebacin-, Decandicarbon-, Phthal-, Terephthal-, Isophthal- Tetrahydrophthal- oder Trimellithsäure sowie Säureanhydride wie Phthal-, Trimellith- oder Bernsteinsäureanhydrid oder deren beliebige Gemische untereinander. Die Polyesterpolyole können auch auf natürlichen Rohstoffen wie Rizinusöl basieren. Es ist ebenfalls möglich, dass die Polyesterpolyole auf Homo- oder Mischpolymerisaten von Lactonen basieren, die bevorzugt durch Anlagerung von Lactonen bzw. Lactongemischen wie Butyrolacton, ε-Caprolacton und / oder Methyl-ε-caprolacton an hydroxyfunktionelle Verbindungen wie mehrwertige Alkohole einer OH-Funktionalität ≥ 2 beispielsweise der nachstehend genannten Art erhalten werden können.

Beispiele für geeignete Alkohole sind alle mehrwertigen Alkohole wie z.B. die C₂ - C₁₂-Diole, die isomeren Cyclohexandiole, Glycerin oder deren beliebige Gemische untereinander.

Geeignete Polycarbonatpolyole sind in an sich bekannter Weise durch Umsetzung von organischen Carbonaten oder Phosgen mit Diolen oder Diol-Mischungen zugänglich.

Geeignete organische Carbonate sind Dimethyl-, Diethyl- und Diphenylcarbonat.

Geeignete Diole bzw. Mischungen umfassen die an sich im Rahmen der Polyestersegmente genannten mehrwertigen Alkohole einer OH-Funktionalität ≥ 2, bevorzugt Butandiol-1,4, Hexandiol-1,6 und / oder 3-Methylpentandiol. Auch Polyesterpolyole können zu Polycarbonatpolyolen umgearbeitet werden.

Geeignete Polyetherpolyole sind gegebenenfalls blockweise aufgebaute Polyadditionsprodukte cyclischer Ether an OH- oder NH-funktionelle Startermoleküle.

Geeignete cyclische Ether sind beispielsweise Styroloxide, Ethylenoxid, Propylenoxid, Tetrahydrofuran, Butylenoxid, Epichlorhydrin sowie ihre beliebigen Mischungen.

Als Starter können die an sich im Rahmen der Polyesterpolyole genannten mehrwertigen Alkohole einer OH-Funktionalität ≥ 2 sowie primäre oder sekundäre Amine und Aminoalkohole verwendet werden.

Bevorzugte Polyetherpolyole sind solche der vorgenannten Art ausschließlich basierend auf Propylenoxid oder statistische oder Block-Copolymere basierend auf Propylenoxid mit weiteren 1-Alkylenoxiden. Besonders bevorzugt sind Propylenoxid-homopolymere sowie statistische oder Block-Copolymere, die Oxyethylen-, Oxypropylen- und / oder Oxybutyleneinheiten aufweisen, wobei der Anteil der Oxypropyleneinheiten bezogen auf die Gesamtmenge aller Oxyethylen-, Oxypropylen-und Oxybutyleneinheiten mindestens 20 Gew.-%, bevorzugt mindestens 45 Gew.-% ausmacht. Oxypropylen- und Oxybutylen umfasst hierbei alle jeweiligen linearen und verzweigten C₃- und C₄-Isomere.

Daneben sind als Bestandteile der Polyol-Komponente b1) als polyfunktionelle, isocyanatreaktive Verbindungen auch niedermolekulare, d.h. mit Molekulargewichten ≤ 500 g/mol, kurzkettige, d.h. 2 bis 20 Kohlenstoffatome enthaltende aliphatische, araliphatische oder cycloaliphatische di-, tri- oder polyfunktionelle Alkohole geeignet.

Dies können beispielsweise in Ergänzung zu den oben genannten Verbindungen Neopentylglykol, 2-Ethyl-2-butylpropandiol, Trimethylpentandiol, stellungs-isomere Diethyloctandiole, Cyclohexandiol, 1,4-Cyclohexandimethanol, 1,6-Hexandiol, 1,2- und 1,4-Cyclohexandiol, hydriertes Bisphenol A, 2,2-Bis(4-hydroxy-cyclohexyl)-propan oder 2,2-Dimethyl-3-hydroxypropionsäure, 2,2-dimethyl-3-hydroxypropyl-ester sein. Beispiele geeigneter Triole sind Trimethylolethan, Trimethylolpropan oder Glycerin. Geeignete höherfunktionelle Alkohole sind Di-(trimethylolpropan), Pentaerythrit, Dipentaerythrit oder Sorbit.

Besonders bevorzugt ist, wenn die Polyolkomponente ein difunktioneller Polyether-, Polyester oder ein Polyether-polyester-block-copolyester oder ein Polyether-Polyester-Blockcopolymer mit primären OH-Funktionen ist.

Es ist ebenfalls möglich, als isocyanatreaktive Verbindungen b1) Amine einzusetzen. Beispiele geeigneter Amine sind Ethylendiamin, Propylendiamin, Diaminocyclohexan, 4,4'-Dicylohexylmethandiamin, Isophorondiamin (IPDA), difunktionelle Polyamine wie z.B. die Jeffamine®, aminterminierte Polymere, insbesondere mit zahlenmittleren Molmassen ≤ 10000 g/Mol. Mischungen der vorgenannten Amine können ebenfalls verwendet werden.

Es ist ebenfalls möglich, als isocyanatreaktive Verbindungen b1) Aminoalkohole einzusetzen. Beispiele geeigneter Aminoalkohole sind die isomeren Aminoethanole, die isomere Aminopropanole die isomeren Aminobutanole und die isomeren Aminohexanole oder deren beliebige Mischungen.

Alle vorgenannten isocyanatreaktiven Verbindungen b1) können untereinander beliebig vermischt werden.

Bevorzugt ist auch, wenn die isocyanatreaktiven Verbindungen b1) eine zahlenmittlere Molmasse von ≥ 200 und ≤ 10000 g/Mol, weiter bevorzugt ≥ 500 und ≤ 8000 g/Mol und ganz besonders bevorzugt ≥ 800 und ≤ 5000 g/Mol aufweisen. Die OH-Funktionalität der Polyole beträgt bevorzugt 1.5 bis 6.0, besonders bevorzugt 1.8 bis 4.0.

Die Prepolymere der Polyisocyanat-Komponente a) können insbesondere einen Restgehalt an freiem monomeren Di- und Triisocyanaten < 1 Gew.-%, besonders bevorzugt < 0.5 Gew.-% und ganz besonders bevorzugt < 0.3 Gew.-% aufweisen.

Es ist gegebenenfalls auch möglich, dass die Polyisocyanat-Komponente a) vollständig oder anteilsmäßig organische Verbindung enthält, deren NCO-Gruppen ganz oder teilweise mit aus der Beschichtungstechnologie bekannten Blockierungsmitteln umgesetzt sind. Beispiel für Blockierungsmittel sind Alkohole, Lactame, Oxime, Malonester, Pyrazole sowie Amine, wie z.B. Butanonoxim, Diisopropylamin, Malonsäurediethylester, Acetessigester, 3,5-Dimethylpyrazol, ε-Caprolactam, oder deren Mischungen.

Besonders bevorzugt ist, wenn die Polyisocyanat-Komponente a) Verbindungen mit aliphatisch gebundenen NCO-Gruppen umfasst, wobei unter aliphatisch gebundenen NCO-Gruppen derartige Gruppen verstanden werden, die an ein primäres C-Atom gebunden sind. Die isocyanatreaktive Komponente b) umfasst bevorzugt wenigstens eine organische Verbindung, die im Mittel wenigstens 1.5 und bevorzugt 2 bis 3 isocyanatreaktive Gruppen aufweist. Im Rahmen der vorliegenden Erfindung werden als isocyanatreaktive Gruppen bevorzugt Hydroxy-, Amino- oder Mercapto-Gruppen angesehen.

Die isocyanatreaktive Komponente kann insbesondere Verbindungen umfassen, die im Zahlenmittel wenigstens 1.5 und bevorzugt 2 bis 3 isocyanatreaktive Gruppen aufweisen.

Geeignete polyfunktionelle, isocyanatreaktive Verbindungen der Komponente b) sind beispielsweise die oben beschriebenen Verbindungen b1).

Bei einer weiteren bevorzugten Ausführungsform ist vorgesehen, dass das Schreibmonomer c) wenigstens ein mono- und / oder ein multifunktionales Schreibmonomer umfasst oder daraus besteht. Weiter bevorzugt kann das Schreibmonomer wenigstens ein mono- und / oder ein multifunktionelles (Meth)acrylat-Schreibmonomer umfassen oder daraus bestehen. Ganz besonders bevorzugt kann das Schreibmonomer wenigstens ein mono- und / oder ein multifunktionelles Urethan(meth)acrylat umfassen oder daraus bestehen.

Geeignete Acrylat-Schreibmonomere sind insbesondere Verbindungen der allgemeinen Formel (VI) 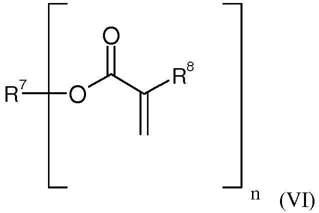 bei denen n≥1 und n≤4 ist und R⁷ ein linearer, verzweigter, cyclischer oder heterocyclischer unsubstituierter oder gegebenenfalls auch mit Heteroatomen substituierter organischer Rest und/oder R⁸ Wasserstoff, ein linearer, verzweigter, cyclischer oder heterocyclischer unsubstituierter oder gegebenenfalls auch mit Heteroatomen substituierter organischer Rest ist. Besonders bevorzugt ist R⁸ Wasserstoff oder Methyl und/oder R⁷ ein linearer, verzweigter, cyclischer oder heterocyclischer unsubstituierter oder gegebenenfalls auch mit Heteroatomen substituierter organischer Rest.

Als Acrylate bzw. Methacrylate werden vorliegend Ester der Acrylsäure bzw. Methacrylsäure bezeichnet. Beispiele bevorzugt verwendbarer Acrylate und Methacrylate sind Phenylacrylat, Phenylmethacrylat, Phenoxyethylacrylat, Phenoxyethylmethacrylat, Phenoxyethoxyethylacrylat, Phenoxyethoxyethylmethacrylat, Phenylthioethylacrylat, Phenylthioethylmethacrylat, 2-Naphthylacrylat, 2-Naphthylmethacrylat, 1,4-Bis-(2-thionaphthyl)-2-butylacrylat, 1,4-Bis-(2-thionaphthyl)-2-butylmethacrylat, Bisphenol A Diacrylat, Bisphenol A Dimethacrylat, sowie deren ethoxylierte Analogverbindungen, N-Carbazolylacrylate.

Als Urethanacrylate werden vorliegend Verbindungen mit mindestens einer Acrylsäureestergruppe und mindestens eine Urethanbindung verstanden. Solche Verbindungen können beispielsweise durch Umsetzung eines Hydroxy-funktionellen Acrylats oder Methacrylats mit einer Isocyanat-funktionellen Verbindung erhalten werden.

Beispiele hierfür verwendbarer Isocyanat-funktionelle Verbindungen sind Monoisocyanate sowie die unter a) genannten monomeren Diisocyanate, Triisocyanate und / oder Polyisocyanate. Beispiele geeigneter Monoisocyanate sind Phenylisocyanat, die isomeren Methylthiophenylisocyanate. Di-, Tri-oder Polyisocyanate sind oben genannt sowie Triphenylmethan-4,4',4"-triisocyanat und Tris(p-isocyanatophenyl)thiophosphat oder deren Derivate mit Urethan-, Harnstoff-, Carbodiimid-, Acylharnstoff-, Isocyanurat-, Allophanat-, Biuret-, Oxadiazintrion-, Uretdion-, Iminooxadiazindionstruktur und Mischungen derselben. Bevorzugt sind dabei aromatische Di-, Tri- oder Polyisocyanate.

Als hydroxyfunktionelle Acrylate oder Methacrylate für die Herstellung von Urethanacrylaten kommen beispielsweise Verbindungen wie 2-Hydroxyethyl(meth)acrylat, Polyethylenoxid-mono-(meth)acrylate, Polypropylenoxidmono(meth)acrylate, Polyalkylenoxidmono(meth)-acrylate, Poly(ε-caprolacton)mono(meth)acrylate, wie z.B. Tone® M100 (Dow, Schwalbach, DE), 2-Hydroxypropyl-(meth)acrylat, 4-Hydroxybutyl(meth)acrylat, 3-Hydroxy-2,2-dimethylpropyl-(meth)acrylat, Hydroxypropyl(meth)acrylat, Acrylsäure-(2-hydroxy-3-phenoxypropylester), die hydroxyfunktionellen Mono-, Di- oder Tetraacrylate mehrwertiger Alkohole wie Trimethylolpropan, Glycerin, Pentaerythrit, Dipentaerythrit, ethoxyliertes, propoxyliertes oder alkoxyliertes Trimethylolpropan, Glycerin, Pentaerythrit, Dipentaerythrit oder deren technische Gemische. Bevorzugt sind 2-Hydroxyethylacrylat, Hydroxypropylacrylat, 4-Hydroxybutylacrylat und Poly(ε-caprolacton)mono(meth)acrylat.

Ebenfalls verwendet werden können die an sich bekannten hydroxylgruppenhaltigen Epoxy(meth)acrylate mit OH-Gehalten von 20 bis 300 mg KOH/g oder hydroxylgruppenhaltige Polyurethan(meth)acrylate mit OH-Gehalten von 20 bis 300 mg KOH/g oder acrylierte Polyacrylate mit OH-Gehalten von 20 bis 300 mg KOH/g sowie deren Mischungen untereinander und Mischungen mit hydroxylgruppenhaltigen ungesättigten Polyestern sowie Mischungen mit Polyester(meth)acrylaten oder Mischungen hydroxylgruppenhaltiger ungesättigter Polyester mit Polyester(meth)acrylaten.

Bevorzugt sind insbesondere Urethanacrylate erhältlich aus der Umsetzung von Tris(p-isocyanatophenyl)thiophosphat und / oder m-Methylthiophenylisocyanat mit alkoholfunktionellen Acrylaten wie Hydroxyethyl(meth)acrylat, Hydroxypropyl(meth)acrylat und / oder Hydroxybutyl(meth)acrylat.

Ebenso ist es möglich, dass das Schreibmonomer weitere ungesättigte Verbindungen wie α,β-ungesättigte Carbonsäurederivate wie beispielsweise Maleinate, Fumarate, Maleimide, Acrylamide, weiterhin Vinylether, Propenylether, Allylether und Dicyclopentadienyl-Einheiten enthaltende Verbindungen sowie olefinisch ungesättigte Verbindungen wie z.B. Styrol, α-Methylstyrol, Vinyltoluol und / oder Olefine, umfasst oder daraus besteht.

Photoinitiatoren der Komponente d) sind üblicherweise durch aktinische Strahlung aktivierbare Verbindungen, die eine Polymerisation der Schreibmonomere auslösen können. Bei den Photoinitiatoren kann zwischen unimolekularen (Typ I) und bimolekularen (Typ II) Initiatoren unterschieden werden.

Des Weiteren werden sie je nach ihrer chemischen Natur in Photoinitiatoren für radikalische, anionische, kationische oder gemischte Art der Polymerisation unterschieden.
Typ I-Photoinitiatoren (Norrish-Typ-I) für die radikalische Photopolymerisation bilden beim Bestrahlen durch eine unimolekulare Bindungsspaltung freie Radikale. Beispiele für Typ I-Photoinitiatoren sind Triazine, Oxime, Benzoinether, Benzilketale, Bis-imidazole, Aroylphosphinoxide, Sulfoniumund Iodoniumsalze.
Typ II-Photoinitiatoren (Norrish-Typ-II) für die radikalische Polymerisation bestehen aus einem Farbstoff als Sensibilisator und einem Coinitiator und durchlaufen bei der Bestrahlung mit auf den Farbstoff angepasstem Licht eine bimolekulare Reaktion. Zunächst absorbiert der Farbstoff ein Photon und überträgt aus einem angeregten Zustand Energie auf den Coinitiator. Dieser setzt durch Elektronen-oder Protonentransfer oder direkte Wasserstoffabstraktion die polymerisationsauslösenden Radikale frei.

Im Sinne dieser Erfindung werden bevorzugt Typ II-Photoinitiatoren verwendet.

Solche Photoinitiatorsysteme sind prinzipiell in der EP 0 223 587 A beschriebenen und bestehen bevorzugt aus einer Mischung von einem oder mehreren Farbstoffen mit Ammoniumalkylarylborat(en).

Geeignete Farbstoffe, die zusammen mit einem Ammoniumalkylarylborat einen Typ II-Photoinitiator bilden, sind die in der WO 2012062655 beschriebenen kationischen Farbstoffe in Kombination mit den eben dort beschriebenen Anionen.

Unter kationischen Farbstoffen werden bevorzugt solche der folgenden Klassen verstanden: AcridinFarbstoffe, Xanthen-Farbstoffe, Thioxanthen-Farbstoffe, Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, extern kationische Merocyanin-Farbstoffe, extern kationische Neutrocyanin-Farbstoffe, Nullmethin-Farbstoffe - insbesondere Naphtholactam-Farbstoffe, Streptocyanin-Farbstoffe. Solche Farbstoffe sind beispielsweise in H. Berneth in Ullmann's Encyclopedia of Industrial Chemistry, Azine Dyes, Wiley-VCH Verlag, 2008, H. Berneth in Ullmann's Encyclopedia of Industrial Chemistry, Methine Dyes and Pigments, Wiley-VCH Verlag, 2008, T. Gessner, U. Mayer in Ullmann's Encyclopedia of Industrial Chemistry, Triarylmethane and Diarylmethane Dyes, Wiley-VCH Verlag, 2000 beschrieben.

Besonders bevorzugt sind Phenazin-Farbstoffe, Phenoxazin-Farbstoffe, Phenothiazin-Farbstoffe, Tri(het)arylmethan-Farbstoffe - insbesondere Diamino- und Triamino(het)arylmethan-Farbstoffe, Mono-, Di-, Tri- und Pentamethincyanin-Farbstoffe, Hemicyanin-Farbstoffe, Nullmethin-Farbstoffe - insbesondere Naphtholactam-Farbstoffe, Streptocyanin-Farbstoffe.

Beispiele für kationische Farbstoffe sind Astrazon Orange G, Basic Blue 3, Basic Orange 22, Basic Red 13, Basic Violett 7, Methylenblau, Neu Methylenblau, Azur A, 2,4-Diphenyl-6-(4-methoxyphenyl)pyrylium, Safranin O, Astraphloxin, Brilliant Grün, Kristallviolett, Ethylviolett und Thionin.

Bevorzugte Anionen sind insbesondere C₈- bis C₂₅-Alkansulfonat, vorzugsweise C₁₃- bis C₂₅-Alkansulfonat, C₃- bis C₁₈-Perfluoralkansulfonat, C₄- bis C₁₈-Perfluoralkansulfonat, das in der Alkylkette mindestens 3 Wasserstoffatome trägt, C₉- bis C₂₅-Alkanoat, C₉- bis C₂₅-Alkenoat, Cs- bis C₂₅-Alkylsulfat, vorzugsweise C₁₃- bis C₂₅-Alkylsulfat, C₈- bis C₂₅-Alkenylsulfat, vorzugsweise C₁₃- bis C₂₅-Alkenylsulfat, C₃- bis C₁₈-Perfluoralkylsulfat, C₄- bis C₁₈-Perfluoralkylsulfat, das in der Alkylkette mindestens 3 Wasserstoffatome trägt, Polyethersulfate basierend auf mindestens 4 Äquivalenten Ethylenoxid und/oder 4 Äquivalenten Propylenoxid, Bis-C₄- bis C₂₅-Alkyl-, C₅- bis C₇-Cycloalkyl-, C₃- bis C₈-Alkenyl- oder C₇- bis C₁₁-Aralkyl-sulfosuccinat, durch mindestens 8 Fluoratome substituiertes Bis-C₂- bis C₁₀-alkyl-sulfosuccinat, C₈- bis C₂₅-Alkyl-sulfoacetate, durch mindestens einen Rest der Gruppe Halogen, C₄- bis C₂₅-Alkyl, Perfluor-C₁- bis C₈-Alkyl und/oder C₁- bis C₁₂-Alkoxycarbonyl substituiertes Benzolsulfonat, ggf. durch Nitro, Cyano, Hydroxy, C₁- bis C₂₅-Alkyl, C₁- bis C₁₂-Alkoxy, Amino, C₁- bis C₁₂-Alkoxycarbonyl oder Chlor substituiertes Naphthalin- oder Biphenylsulfonat, ggf. durch Nitro, Cyano, Hydroxy, C₁- bis C₂₅-Alkyl, C₁- bis C₁₂-Alkoxy, C₁- bis C₁₂-Alkoxycarbonyl oder Chlor substituiertes Benzol-, Naphthalin- oder Biphenyldisulfonat, durch Dinitro, C₆- bis C₂₅-Alkyl, C₄- bis C₁₂-Alkoxycarbonyl, Benzoyl, Chlorbenzoyl oder Toluoyl substituiertes Benzoat, das Anion der Naphthalindicarbonsäure, Diphenyletherdisulfonat, sulfonierte oder sulfatierte, ggf. mindestens einfach ungesättigte C₈- bis C₂₅-Fettsäureester von aliphatischen C₁- bis C₈-Alkoholen oder Glycerin, Bis-(sulfo-C₂- bis C₆-alkyl)-C₃- bis C₁₂-alkandicarbonsäureester, Bis-(sulfo-C₂- bis C₆-alkyl)-itaconsäureester, (Sulfo-C₂- bis C₆-alkyl)-C₆- bis C₁₈-alkancarbonsäureester, (Sulfo-C₂- bis C₆-alkyl)-acryl- oder methacrylsäureester, ggf. durch bis zu 12 Halogenreste substituiertes Triscatecholphosphat, ein Anion der Gruppe Tetraphenylborat, Cyanotriphenylborat, Tetraphenoxyborat, C₄- bis C₁₂-Alkyl-triphenylborat, deren Phenyl- oder Phenoxy-Reste durch Halogen, C₁- bis C₄-Alkyl und/oder C₁- bis C₄-Alkoxy substituiert sein können, C₄- bis C₁₂-Alkyl-trinaphthylborat, Tetra-Ci- bis C₂₀-alkoxyborat, 7,8- oder 7,9-Dicarba-nido-undecaborat(1-) oder (2-), die gegebenenfalls an den B- und/oder C-Atomen durch eine oder zwei C₁- bis C₁₂-Alkyl- oder Phenyl-Gruppen substituiert sind, Dodecahydro-dicarbadodecaborat(2-) oder B-C₁- bis C₁₂-Alkyl-C-phenyl-dodecahydro-dicarbadodecaborat(1-) steht, wobei bei mehrwertigen Anionen wie Naphthalindisulfonat A⁻ für ein Äquivalent dieses Anions steht, und wobei die Alkan- und Alkylgruppen verzweigt sein können und/oder durch Halogen, Cyano, Methoxy, Ethoxy, Methoxycarbonyl oder Ethoxycarbonyl substituiert sein können.

Bevorzugt ist auch, wenn das Anion A⁻ des Farbstoffs einen AClogP im Bereich von 1 bis 30, besonders bevorzugt im Bereich von 1 bis 12 und insbesondere bevorzugt im Bereich von 1 bis 6,5 aufweist. Der AClogP wird nach J. Comput. Aid. Mol. Des. 2005, 19, 453; Virtual Computational Chemistry Laboratory, http://www.vcclab.org berechnet.

Geeignete Ammoniumalkylarylborate sind beispielsweise (Cunningham et al., RadTech'98 North America UV/EB Conference Proceedings, Chicago, Apr. 19-22, 1998): Tetrabutylammonium Triphenylhexylborat, Tetrabutylammonium Triphenylbutylborat, Tetrabutylammonium Trinapthylhexylborat, Tetrabutylammonium Tris(4-tert.butyl)-phenylbutylborat, Tetrabutylammonium Tris-(3-fluorphenyl)-hexylborat hexylborat ([191726-69-9], CGI 7460, Produkt der BASF SE, Basel, Schweiz), 1-Methyl-3-octylimidazolium Dipentyldiphenylborat und Tetrabutylammonium Tris-(3-chlor-4-methylphenyl)-hexylborat ([1147315-11-4], CGI 909, Produkt der BASF SE, Basel, Schweiz).

Es kann vorteilhaft sein, Gemische dieser Photoinitiatoren einzusetzen. Je nach verwendeter Strahlungsquelle muss Typ und Konzentration an Photoinitiator in dem Fachmann bekannter Weise angepasst werden. Näheres ist zum Beispiel in P. K. T. Oldring (Ed.), Chemistry & Technology of UV & EB Formulations For Coatings, Inks & Paints, Vol. 3, 1991, SITA Technology, London, S. 61 - 328 beschrieben.

Ganz besonders bevorzugt ist, wenn der Photoinitiator eine Kombination von Farbstoffen, deren Absorptionsspektren zumindest teilweise den Spektralbereich von 400 bis 800 nm abdecken, mit wenigstens einem auf die Farbstoffe abgestimmten Coinitiator umfasst.

Bevorzugt ist auch, wenn wenigstens ein für eine Laserlichtfarbe ausgewählt aus blau, gelb, grün und rot geeigneter Photoinitiator in der Photopolymer-Formulierung enthalten ist.

Weiter bevorzugt ist auch, wenn die Photopolymer-Formulierung für wenigstens zwei Laserlichtfarben ausgewählt aus blau, grün und rot je einen geeigneten Photoinitiator enthält.

Ganz besonders bevorzugt ist schließlich, wenn die Photopolymer-Formulierung für jede der Laserlichtfarben blau, grün und rot jeweils einen geeigneten Photoinitiator enthält.

Gemäß einer weiteren bevorzugten Ausführungsform ist vorgesehen, dass die Photopolymer-Formulierung zusätzlich Urethane als Additive enthält, wobei die Urethane insbesondere mit wenigstens einem Fluoratom substituiert sein können.

Bevorzugt können die Urethane die allgemeine Formel (VII) 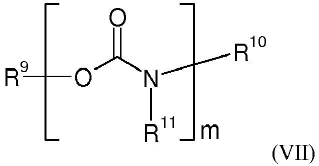 haben, in der m≥1 und m≤8 ist und R⁹, R¹⁰ und R¹¹ lineare, verzweigte, cyclische oder heterocyclische unsubstituierte oder gegebenenfalls auch mit Heteroatomen substituierte organische Reste und/oder R¹⁰, R¹¹ unabhängig voneinander Wasserstoff sind, wobei bevorzugt mindestens einer der Reste R⁹, R¹⁰, R¹¹ mit wenigstens einem Fluoratom substituiert ist und besonders bevorzugt R⁹ ein organischer Rest mit mindestens einem Fluoratom ist. Besonders bevorzugt ist R¹⁰ ein linearer, verzweigter, cyclischer oder heterocyclischer unsubstituierter oder gegebenenfalls auch mit Heteroatomen wie beispielsweise Fluor substituierter organischer Rest.

Ein weiterer Gegenstand der vorliegenden Erfindung ist ein Photopolymer enthaltend eine Phoropolymer-Formulierung, insbesondere umfassend Matrixpolymere, ein Schreibmonomer und einen Photoinitiatorsystem, das zusätzlich eine Verbindung der Formel (I) umfasst.

Die Matrixpolymere des erfindungsgemäßen Photopolymers können insbesondere vernetzt und besonders bevorzugt dreidimensional vernetzt sein.

Vorteilhaft ist auch, wenn die Matrixpolymere Polyurethane sind, wobei die die Polyurethane insbesondere durch Umsetzung wenigstens einer Polyisocyanat-Komponente mit wenigstens einer Isocyanat-reaktiven Komponente erhältlich sein können.

Die oben zu der erfindungsgemäßen Photopolymer-Formulierung getroffenen Aussagen bezüglich weiterer bevorzugten Ausführungsformen gelten analog auch für das erfindungsgemäße Photopolymer.

Auch offenbart ist auch ein holographisches Medium insbesondere in Form eines Films, enthaltend ein Photopolymer wie offenbart oder erhältlich unter Verwendung einer Photopolymer-Formulierung wie offenbart. Auch offenbart die Verwendung einer Photopolymer-Formulierung, wie offenbart, zur Herstellung holographischer Medien.

Bei einer bevorzugten Ausführungsform des holographisches Mediums sind in dieses holgraphische Informationen einbelichtet.

Die holographischen Medien können durch entsprechende Belichtungsprozesse für optische Anwendungen im gesamten sichtbaren und nahen UV-Bereich (300-800 nm) zu Hologrammen verarbeitet werden können. Visuelle Hologramme umfassen alle Hologramme, die nach dem Fachmann bekannten Verfahren aufgezeichnet werden können. Darunter fallen unter anderem In-Line (Gabor) Hologramme, Off-Axis Hologramme, Full-Aperture Transfer Hologramme, Weißlicht-Transmissionshologramme ("Regenbogenhologramme), Denisyukhologramme, Off-Axis Reflektionshologramme, Edge-Lit Hologramme sowie holographische Stereogramme. Bevorzugt sind Reflektionshologramme, Denisyukhologramme, Transmissionshologramme.

Mögliche optische Funktionen der Hologramme, die mit den erfindungsgemäßen Photopolymer-Formulierungen hergestellt werden können entsprechen den optische Funktionen von Lichtelementen wie Linsen, Spiegel, Umlenkspiegel, Filter, Streuscheiben, Beugungselemente, Diffusoren, Lichtleiter, Lichtlenker (waveguides), Projektionsscheiben und/oder Masken. Ebenfalls können Kombinationen aus dies optischen Funktionen unabhängig voneinander in einem Hologramm vereinigt sein. Häufig zeigen diese optischen Elemente eine Frequenzselektivität, je nachdem wie die Hologramme belichtet wurden und welche Dimensionen das Hologramm hat.

Zudem können mittels der offenbarten Medien auch holographische Bilder oder Darstellungen hergestellt werden, wie zum Beispiel für persönliche Portraits, biometrische Darstellungen in Sicherheitsdokumenten, oder allgemein von Bilder oder Bildstrukturen für Werbung, Sicherheitslabels, Markenschutz, Markenbranding, Etiketten, Designelementen, Dekorationen, Illustrationen, Sammelkarten, Bilder und dergleichen sowie Bilder, die digitale Daten repräsentieren können u.a. auch in Kombination mit den zuvor dargestellten Produkten. Holographische Bilder können den Eindruck eines dreidimensionalen Bildes haben, sie können aber auch Bildsequenzen, kurze Filme oder eine Anzahl von verschiedenen Objekten darstellen, je nachdem aus welchem Winkel, mit welcher (auch bewegten) Lichtquelle etc. diese beleuchtet wird. Aufgrund dieser vielfältigen Design-Möglichkeiten stellen Hologramme, insbesondere Volumenhologramme, eine attraktive technische Lösung für die oben genannten Anwendung dar.

Ebenfalls offenbart ist daher die Verwendung eines holographischen Mediums zur Aufzeichnung von In-Line, Off-Axis, Full-Aperture Transfer, Weißlicht-Transmissions, Denisyuk, Off-Axis Reflektions oder Edge-Lit Hologrammen sowie holographischen Stereogrammen, insbesondere zur Herstellung von optischen Elementen, Bildern oder Bilddarstellungen.

Ebenfalls offenbart sind Hologramme enthaltend das offenbarte holographische Medium.

Ebenfalls offenbart ist ein Verfahren zur Herstellung eines Hologramms, bei dem zur Belichtung des Mediums gepulste Laserstrahlung verwendet wird.

In einer Ausführungsform des offenbarten Verfahrens beträgt die Pulsdauer ≤ 200 ns, bevorzugt ≤ 100 ns, besonders bevorzugt ≤ 60 ns. Die Pulsdauer darf 0.5 ns nicht unterschreiten. Besonders bevorzugt ist eine Pulsdauer von 4 ns.

Weiterhin offenbart ist auch ein Verfahren zur Herstellung eines holographischen Mediums unter Verwendung einer offenbarten Photopolymer-Formulierung.

Die Photopolymer-Formulierungen können insbesondere zur Herstellung holographischer Medien in Form eines Films verwendet werden. Dabei wird als Träger eine Lage eines für Licht im sichtbaren Spektralbereich (Transmission größer als 85% im Wellenlängenbereich von 400 bis 780 nm) transparenten Materials oder Materialverbunds ein- oder beidseitig beschichtet sowie ggf. eine Abdeckschicht auf der oder den Photopolymerlagen appliziert.

Bevorzugte Materialien oder Materialverbünde des Trägers basieren auf Polycarbonat (PC), Polyethylenterephthalat (PET), Polybutylenterephthalat, Polyethylen, Polypropylen, Celluloseacetat, Cellulosehydrat, Cellulosenitrat, Cycloolefinpolymere, Polystyrol, Polyepoxide, Polysulfon, Cellulosetriacetat (CTA), Polyamid, Polymethylmethacrylat, Polyvinylchlorid, Polyvinylbutyral oder Polydicyclopentadien oder deren Mischungen. Besonders bevorzugt basieren sie auf PC, PET und CTA. Materialverbünde können Folienlaminate oder Coextrudate sein. Bevorzugte Materialverbünde sind Duplex- und Triplexfolien aufgebaut nach einem der Schemata A/B, A/B/A oder A/B/C. Besonders bevorzugt sind PC/PET, PET/PC/PET und PC/TPU (TPU = Thermoplastisches Polyurethan).

Die Materialien oder Materialverbünde des Trägers können einseitig oder beidseitig antihaftend, antistatisch, hydrophobiert oder hydrophiliert ausgerüstet sein. Die genannten Modifikationen dienen an der Photopolymerschicht zugewandten Seite dem Zweck, dass die Photopolymerlage von dem Träger zerstörungsfrei abgelöst werden kann. Eine Modifikation der der Photopolymerlage abgewandten Seite des Trägers dient dazu, dass die erfindungsgemäßen Medien speziellen mechanischen Anforderungen genügen, die z.B. bei der Verarbeitung in Rollenlaminatoren, insbesondere bei Rolle-zu-Rolle-Verfahren, gefordert sind.

Die folgenden Beispiele dienen der beispielhaften Erläuterung der Erfindung ohne sie auf diese zu beschränken.

### Beispiele

### Messmethoden:

### OH Zahl:

Die angegebenen OH-Zahlen wurden gemäß DIN 53240-2 bestimmt.

### NCO-Wert:

Die angegebenen NCO-Werte (Isocyanat-Gehalte) wurden gemäß DIN EN ISO 11909 bestimmt.

### Bestimmung der Kopftemperatur:

Die Kopftemperatur wurde mittels eines im Siedegleichgewicht oberhalb einer Füllkörperkolonne eingesetzten PT100 Thermoelements und kalibriertem Messumformer 4-20 mA in Grad Celsius (°C) bestimmt.

### Bestimmung der Innentemperatur:

Die Innentemperatur wurde mittels eines im Reaktionsraum eingesetzten PT100 Thermoelements und kalibriertem Messumformer 4-20 mA in Grad Celsius (°C) bestimmt.

### Bestimmung der Beugungseffizienz bei Puls-Belichtung:

Zur Bestimmung der Beugungseffizienz bei Puls-Belichtung wurde das Denisyuk Hologramm eines Spiegels in eine Probe, bestehend aus einer Glasplatte mit auflaminiertem Photopolymerfilm, aufgezeichnet. Das Substrat des Photopolymerfilms zeigte dabei zur Laserquelle und das Glassubstrat zum Spiegel. Die Probe war bei der Belichtung mit ihrer Planfläche senkrecht zum Laserstrahl ausgerichtet. Der Abstand Probe zum Spiegel betrug 3 cm.

Als Laser wurde ein Puls-Laser der Firma Quantel aus Frankreich, Modell Brilliant b, verwendet. Es handelte sich dabei um einen Q-switched Nd-YAG Laser mit einem Modul zur Frequenzverdopplung zu 532 nm. Der Single Frequency Mode wurde durch einen Seed Laser garantiert. Die rechnerische Kohärenzlänge betrug ca. 1 m. Die Pulsdauer 4 ns und die mittlere Ausgangsleistung 3 Watt bei einer Pulswiederholungsrate von 10 Hz.

Mit dem elektronisch geregelten Shutter wurde eine Einfachpulsbelichtung sichergestellt. Die Wellenplatte erlaubte die Rotation der Polarisationsebene des Laserlichts und mit dem darauffolgenden Polarisator wurde der S-polarisierte Anteil des Laserlichtes in Richtung der Probe reflektiert. Die Strahlaufweitung erlaubte die Einstellung der belichteten Fläche. Die Wellenplatte und die Strahlaufweitung wurden so eingestellt, dass die Probe eine Belichtungsdosis von 100 mJ/cm²/Puls erreichte.

Zur Bestimmung der Beugungseffizienz wurden die Proben jeweils genau mit einem Puls belichtet. Nach der Belichtung wurde die Probe auf einem Leuchttisch geblichen.

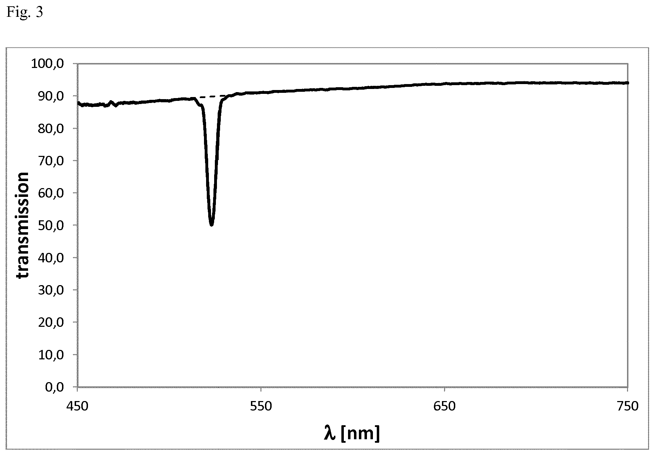

Durch das Hologramm der geblichenen Probe wurde ein Transmissionsspektrum gemessen. Es wurde ein Spektrometer der Firma Ocean Optics, Modell HR4000 verwendet. Die Probe wurde senkrecht zum Lichtstrahl gestellt. Das Transmissionsspektrum zeigte bei der Wellenlänge, bei der die Bragg-Bedingung erfüllt war, einen Einbruch der Transmission. Die Tiefe des Transmissionseinbruchs zur Basislinie wurde als Beugungseffizienz DE des Denisyuk Hologramms des Spiegels ausgewertet (Figur 3).

### Substanzen:

Die verwendeten Lösungsmittel wurden im Chemikalienhandel bezogen.

| | |
|---|---|
| Desmorapid Z | Dibutylzinn-dilaurat [77-58-7], Produkt der Bayer MaterialScience AG, Leverkusen, Deutschland. |
| Desmodur® N 3900 | Produkt der Bayer MaterialScience AG, Leverkusen, DE, Hexandiisocyanat-basiertes Polyisocyanat, Anteil an Iminooxadiazindion mindestens 30 %, NCO-Gehalt: 23.5 %. |
| Fomrez UL 28 | Urethanisierungskatalysator, Handelsprodukt der Momentive Performance Chemicals, Wilton, CT, USA. |
| Natrium-bis(2-ethylhexyl)-sulfosuccinat | [45297-26-5] ist bei Aldrich Chemie, Steinheim erhältlich. |

### Verbindung der Formel (I)

### Beispiel 1: 2-Ethylhexyl-4-[4,6-bis(trichlormethyl)-1,3,5-triazin-2-yl]benzoat

### Herstellung von 2-Ethylhexyl-4-cyanbenzoat nach dem erfindungsgemäßen Ein-Topf-Verfahren:

In einen 25 L Doppelmantel-Emaille-Kessel mit Glasaufsatz, mechanischem Rührer, Destillationsaufsatz mit Rückflußteiler und Dosier- sowie Vakuumeinheit wurden in inerter Atmosphäre 10.91 kg 2-Ethylhexanol (1.5 eq., [104-76-7] Aldrich Chemie, Steinheim, Deutschland) bei 25 °C vorgelegt und 9.00 kg 4-Cyano-benzoesäuremethylester (1.0 eq., [1129-35-7] ABCR GmbH & CO. KG, Karlsruhe, Deutschland) zugegeben. Zu dieser Mischung wurden 32 g Titan(IV)-2-Ethylhexoxid (0.001 eq., [1070-10-6] ABCR GmbH & CO. KG, Karlsruhe, Deutschland) gegeben und die Innentemperatur auf 130 °C erhöht und für 2 h gehalten. In dieser Zeit waren ca. 1.2 kg Methanol (ca. 2/3 der theoretischen Menge) destilliert. Die Vorlage wurde geleert und der Druck auf 500 mbar reduziert. Die Innentemperatur wurde auf 135 °C erhöht für weitere 4 h gehalten. Zu diesem Zeitpunkt wurde kein weiteres Methanol aufgefangen und laut gaschromatographischer Untersuchung enthielt das Reaktionsgemisch kein 4-Cyano-benzoesäuremethylester mehr. Die Innentemperatur wurde auf 100 °C gesenkt, die Vorlage entleert und ein Druck von 10 mbar angelegt. Bei einem Druck von 8.8 - 9.6 mbar und einer Kopftemperatur von 65.5 - 68.5 °C wurden 3.14 kg 2-Ethylhexanol aufgefangen. Nach erneutem Leeren der Vorlage wurde die Innentemperatur auf 185 - 190 °C erhöht und ein Druck von 0.25 mbar angelegt. Bei einer Kopftemperatur von 150.0 - 155.0 °C wurden 14.04 kg (96.9 % der Theorie) 2-Ethylhexyl-4-cyanbenzoat als farbloses Öl erhalten.

GC (Verwendete Säule: 60m Optima-5 HT MS, Temperaturprogramm: T_{Start} = 70 °C für 10 min, dann mit 3 °C/min auf 300 °C, 300 °C für 5 min) Integration in Flächenprozent: 0.11 % 2-Ethylhexanol, 99.54 % 2-Ethylhexyl-4-cyanbenzoat.
¹H NMR (400 MHz, CDCl₃) δ 8.19 - 8.08 (m, 2H), 7.79 - 7.71 (m, 2H), 4.35 - 4.18 (m, 2H), 1.81 - 1.65 (hept, *J* = 6.2 Hz, 1H), 1.53 - 1.23 (m, 8H), 1.01 - 0.86 (m, 6H).

### Herstellung von 2-Ethylhexyl-4-[4,6-bis(trichlormethyl)-1,3,5-triazin-2-yl]benzoat

In einem 1L Dreihalskolben wurden 150.2 g 2-Ethylhexyl-4-cyanbenzoat und 334.4 g Trichloracetonitril vorgelegt und bei 0 °C wurden 15.4 g Aluminiumbromid zugegeben. Zu dieser Mischung wurde getrocknetes HCl (g) bis zu Sättigung gegeben und anschließend die Temperatur über 4 h auf 50 °C erhöht und für 2 h gehalten. Das überschüssige HCl (g) wurde mit Stickstoff ausgetrieben und die Reaktionsmischung mit 100 mL Toluol verdünnt und für 24 h auf -20 °C kaltgestellt. Bei dieser Temperatur schied sich als Nebenprodukt gebildetes Tris(trichlormethyl)-1,3,5-triazin quantitativ ab und die überstehende Lösung wurde dekantiert. Das Lösungsmittel wurde im Vakuum entfernt und man erhielt 256 g eines leicht hellgelben Öls.
¹H NMR (400 MHz, CDCl₃) δ 8.81 - 8.71 (m, 2H), 8.28 - 8.20 (m, 2H), 4.36 - 4.25 (m, 2H), 1.81 - 1.70 (dt, *J* = 12.2, 6.1 Hz, 1H), 1.53 - 1.39 (m, 11H), 1.39 - 1.27 (m, 4H), 1.05 - 0.95 (t, *J* = 7.5 Hz, 3H), 0.95 - 0.87 (m, 3H).
¹³C NMR (101 MHz, CDCl₃) δ 175.42, 174.09, 165.72, 137.00, 135.85, 130.15, 129.97, 94.74, 67.98, 38.93, 30.61, 28.99, 26.90, 24.03, 22.96, 14.03, 11.11.

### Herstellung der weiteren Komponenten für die offenbarte Photopolymer-Formulierung:

### Herstellung von Polyol 1:

In einem 1 L Kolben wurden 0.18 g Zinnoctoat, 374.8 g ε-Caprolacton und 374.8 g eines difunktionellen Polytetrahydrofuranpolyetherpolyols (Equivalentgewicht 500 g/Mol OH) vorgelegt und auf 120 °C aufgeheizt und so lange auf dieser Temperatur gehalten, bis der Festgehalt (Anteil der nicht-flüchtigen Bestandteile) bei 99.5 Gew.-% oder darüber lag. Anschließend wurde abgekühlt und das Produkt als wachsiger Feststoff erhalten.

### Herstellung des Urethanacrylats 1 (Schreibmonomer): Phosphorothioyltris(oxybenzol-4,1-diylcarbamoyloxyethan-2,1-diyl)trisacrylat

In einem 500 mL Rundkolben wurden 0.1 g 2,6-Di-tert.-butyl-4-methylphenol, 0.05 g Dibutylzinn-dilaurat sowie und 213.07 g einer 27 %-igen Lösung von Tris(p-isocyanatophenyl)thiophosphat in Ethylacetat (Desmodur® RFE, Produkt der Bayer MaterialScience AG, Leverkusen, Deutschland) vorgelegt und auf 60 °C erwärmt. Anschließend wurden 42.37 g 2-Hydroxyethylacrylat zugetropft und die Mischung weiter auf 60 °C gehalten, bis der Isocyanatgehalt unter 0.1 % gesunken war. Danach wurde abgekühlt und das Ethylacetat im Vakuum vollständig entfernt. Das Produkt wurde als teilkristalliner Feststoff erhalten.

### Herstellung des Urethanacrylats 2 (Schreibmonomer): 2-({[3-(Methylsulfanyl)phenyl]-carbamoyl}oxy)ethylprop-2-enoat

In einem 100 mL Rundkolben wurden 0.02 g 2,6-Di-tert.-butyl-4-methylphenol, 0.01 g Desmorapid Z, 11.7 g 3-(Methylthio)phenylisocyanat [28479-1-8] vorgelegt und auf 60 °C erwärmt. Anschließend wurden 8.2 g 2-Hydroxyethylacrylat zugetropft und die Mischung weiter auf 60 °C gehalten, bis der Isocyanatgehalt unter 0.1 % gesunken war. Danach wurde abgekühlt. Das Produkt wurde als farblose Flüssigkeit erhalten.

### Herstellung des Additivs 1 Bis(2,2,3,3,4,4,5,5,6,6,7,7-dodecafluorheptyl)-(2,2,4-trimethylhexan-1,6-diyl)biscarbamat

In einem 50 mL Rundkolben wurden 0.02 g Desmorapid Z und 3.6 g 2,4,4-Trimethylhexane-1,6-diisocyanat (TMDI) vorgelegt und auf 60 °C erwärmt. Anschließend wurden 11.9 g 2,2,3,3,4,4,5,5,6,6,7,7-Dodecafluorheptan-1-ol zugetropft und die Mischung weiter auf 60 °C gehalten, bis der Isocyanatgehalt unter 0.1 % gesunken war. Danach wurde abgekühlt. Das Produkt wurde als farbloses Öl erhalten.

### Herstellung des Farbstoffs 1:

Analog zu Beispiel 6 der DE 1 073 662 wurden 6.98 g der Cyanmethylenbase der Formel 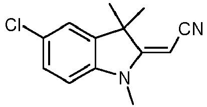 und 6.16 g des Aldehyds der Formel 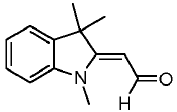 in 30 mL wasserfreiem Toluol langsam unter Rühren mit 3.57 g Thionylchlorid versetzt. Dann wurde 1 h bei 100 °C gerührt und abgekühlt. 50 mL Toluol wurden zugesetzt und der Farbstoff abgesaugt. Dreimal wurde er mit je 30 mL Toluol verrührt und jeweils wieder abgesaugt. Nach Trocknen bei 50 °C im Vakuum wurde der rote Farbstoff wurde in 100 mL Wasser weitgehend gelöst. Eine Lösung von 12.38 g Natrium-bis(2-ethylhexyl)sulfosuccinat in 100 mL Butylacetat wurde hinzugefügt. 1 h wurde das Zweiphasengemisch gerührt und dann in einen Scheidetrichter überführt. Die wässrige Phase wurde abgelassen und die organische Phase viermal mit 40 mL Wasser gewaschen. Nach Abtrennen der letzten Wasserwäsche wurde die organische Phase mit 250 mL Butylacetat verdünnt und am Rotationsverdampfer im Vakuum wasserfrei destilliert. Dabei wurden auch ca. 200 mL Butylacetat abdestilliert, so dass schließlich 150.1 g einer roten Lösung des Farbstoffs der Formel 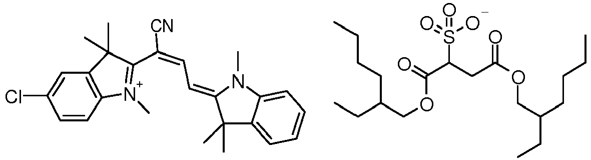 in Butylacetat erhalten wurde, die lagerstabil war.

Eine Probe wurde entnommen und das restliche Lösungsmittel im Vakuum abgezogen. Nach Trocknen bei 50 °C im Vakuum erhielt man den Farbstoff als rote harzige Substanz.

λₘₐₓ (in CH₃CN) = 498 nm und 523 nm, ε = 89580 (bei 498 nm) und 99423 L mol⁻¹ cm⁻¹ (bei 523 nm) L mol⁻¹ cm⁻¹. Mit diesen spektroskopischen Daten konnte die Konzentration der obigen Lösung zu 10.0 % bestimmt werden.

### Herstellung der offenbarten Medien zur Bestimmung der holographischen Eigenschaften

### Herstellung von offenbarten holographischen Medien auf einer Folienbeschichtungsanlage

Im Folgenden wird die kontinuierliche Herstellung von holographischen Medien in der Form von Filmen aus erfindungsgemäßen und nicht erfindungsgemäßen Photopolymer-Formulierungen beschrieben.

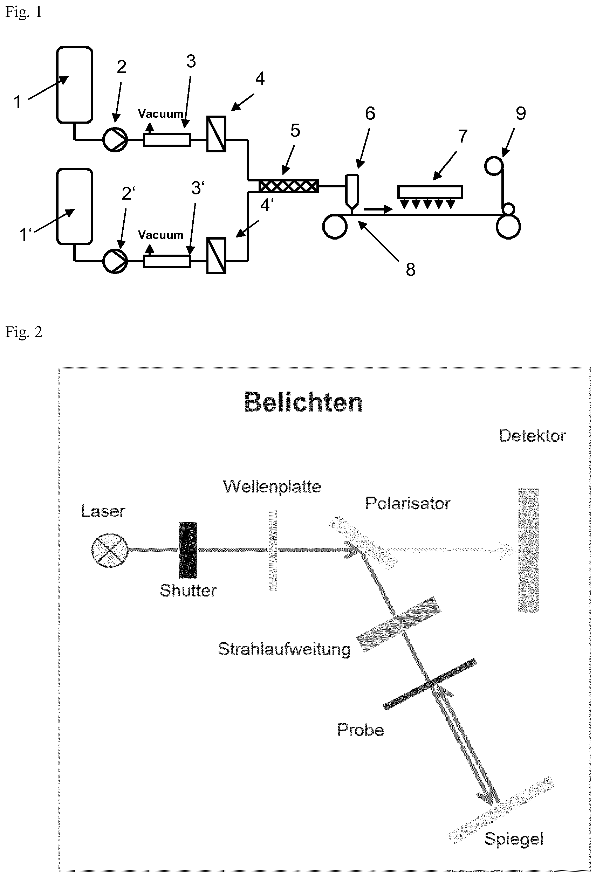

Zur Herstellung wurde die in Fig. 1 dargestellte Folienbeschichtungsanlage verwendet, wobei den einzelnen Bauteilen die folgenden Bezugszeichen zugeordnet sind. Figur 1 zeigt den schematischen Aufbau der verwendeten Beschichtungsanlage. In der Figur haben die einzelnen Bauteile die folgenden Bezugszeichen:
- 1, 1': Vorratsbehälter
- 2, 2': Dosiereinrichtung
- 3, 3': Vakuumentgasungseinrichtung
- 4, 4': Filter
- 5: Statischer Mischer
- 6: Beschichtungseinrichtung
- 7: Umlufttrockner
- 8: Trägersubstrat
- 9: Abdeckschicht

### Beispiel-Medium 1

Zur Herstellung der Photopolymer-Formulierung wurden 39.42 g des Polyols 1 schrittweise mit einer Mischung aus 15.00 g des Urethanacrylats 1 und 15.00 g des Urethanacrylats 2, 11.25 g des Additivs 1, 0.075 g des Beispiels 1, 0.75 g CGI 909, 0.68 g des oberflächenaktiven Additivs BYK® 310, 0.038 g Fomrez UL-28 und 23.65 g Ethylacetat versetzt und gemischt. Anschließend wurden der Mischung 1.50 g einer 10%-igen Lösung des Farbstoffs 1 in Ethylacetat im Dunklen hinzugefügt und vermischt, so dass eine klare Lösung erhalten wurde. Wenn nötig, wurde die Formulierung für kurze Zeit bei 60 °C erwärmt, um die Einsatzstoffe schneller in Lösung zu bringen. Dieses Gemisch wurde in einen der beiden Vorratsbehälter 1 der Beschichtungsanlage eingebracht. In den zweiten Vorratsbehälter 1' wurde die Polyisocyanat-Komponente (Desmodur® N 3900, Handelsprodukt der Bayer MaterialScience AG, Leverkusen, Deutschland, Hexandiisocyanat-basiertes Polyisocyanat, Anteil an Iminooxadiazindion mindestens 30 %, NCO-Gehalt: 23.5 %) eingefüllt. Beide Komponenten wurden dann jeweils durch die Dosiereinrichtungen 2 im Verhältnis von 942.2 (Komponentenmischung) zu 57.8 (Isocyanat) zur Vakuumentgasungseinrichtung 3 gefördert und entgast. Von hier aus wurden sie dann jeweils durch die Filter 4 in den statischen Mischer 5 geleitet, in dem die Vermischung der Komponenten zur Photopolymer-Formulierung erfolgte. Die erhaltene, flüssige Masse wurde dann der Beschichtungseinrichtung 6 zugeführt.

Bei der Beschichtungseinrichtung 6 handelte es sich im vorliegenden Fall um ein dem Fachmann bekanntes Rakelsystem (Doctor Blade). Alternativ kann aber auch eine Schlitzdüse zum Einsatz kommen. Mit Hilfe der Beschichtungseinrichtung 6 wurde die Photopolymer-Formulierung bei einer Verarbeitungstemperatur von 20 °C auf ein Trägersubstrat 8 in der Form einer 36 µm dicken Polyethylenterephthalatfolie appliziert und für 5.8 Minuten bei einer Vernetzungstemperatur von 80 °C in einem Umlufttrockner 7 getrocknet. Dabei wurde ein Medium in der Form eines Films erhalten, der dann mit einer 40 µm dicken Polyethylenfolie als Abdeckschicht 9 versehen und aufgewickelt wurde.

Die gewünschte Zielschichtdicke des Films lag vorzugsweise zwischen 1 und 60 µm, bevorzugt von 5 bis 25 µm, besonders bevorzugt von 10 bis 15 µm.

Die Herstellgeschwindigkeit lag bevorzugt im Bereich von 0.2 m/min bis 300 m/min und besonders bevorzugt im Bereich vom 1.0 m/min bis 50 m/min.

Die erzielte Schichtdicke des Films lag bei 12 µm ± 1 µm.

### Vergleichs-Medium I

Das Vergleich-Medium I wurde in wie oben beschrieben ohne Beispiel 1 hergestellt.

### Holographische Prüfung:

Die wie beschrieben hergestellten Medien wurden mittels einer Messordnung gemäß Figur 2 in der oben beschriebenen Weise (Bestimmung der Beugungseffizienz bei Puls-Belichtung) auf ihre holographischen Eigenschaften geprüft. Dabei ergaben sich folgende Messwerte für DE bei einer fixen Dosis 100 mJ/cm²:

**Tab. 1: Holographische Bewertung ausgewählter Beispiele**

| **Beispiel-Medium** | **Single pulse DE [%]** |
|---|---|
| 1 | 49 |

**Tab. 2: Holographische Bewertung ausgewählter Vergleichs-Medien**

| **Vergleichs-Medium** | **Single pulse DE [%]** |
|---|---|
| I | 2% |

Der gefundene Wert für das Beispiel-Medium 1 zeigt, dass die in den Photopolymer-Formulierungen eingesetzten erfindungsgemäßen Verbindungen der Formel (I) für die Verwendung in holographischen Medien bei Belichtung mit gepulstem Laser sehr gut geeignet sind. Das Vergleichs-Medien I weist keine erfindungsgemäße Verbindung der Formel (I) auf und ist für die Verwendung in holographischen Medien bei Belichtung mit gepulstem Laser ungeeignet.

## Patentansprüche

1. Verfahren zur Herstellung von para-disubstituierten Benzoesäure-nitrilen der Formel (IV) 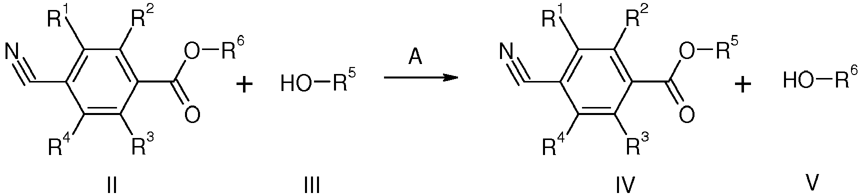 wobei R¹, R², R³ und R⁴ unabhängig voneinander Wasserstoff, Halogen, Alkyl, Alkoxy, Alkenyl, Alkinyl, Alkylthio, Alkylseleno, wobei R¹ und R² und / oder R³ und R⁴ zusammen eine 3 bis 5-gliedrige gesättigte oder ungesättige gegebenenfalls mit bis zu 2 Heteroatomen substituierte Brücke sein können und R⁵ gegebenenfalls beliebig durch Halogen und / oder C₁ bis C₁₀-Alkyl-substituiertes und / oder C₁ bis C₁₀-Alkoxy-substituiertes lineares C₅ bis C₂₀-Alkyl, in dem bis zu 6 Kohlenstoff-Atome durch Sauerstoff ersetzt sein können, wobei zwischen zwei Sauerstoff-Atomen mindestens 2 Kohlenstoff-Atome vorhanden sein müssen und der Rest R⁵ mit mindestens 2 Kohlenstoff-Atomen beginnt, wobei die endständige Methyl-Gruppe des linearen C₅ bis C₂₀-Alkyl-Rests unsubstituiert sein muss, und R6 für Methyl, Ethyl oder isomeres Propyl und A für einen Katalysator A steht, und es sich um ein Ein-Topf-Verfahren handelt, bei dem die Komponente (III) im Überschuss eingesetzt wird, die Komponenten (V), (III) und (IV) in angegebener Reihenfolge in drei Schritten durch Anpassung des Drucks und der Temperatur destillativ abgetrennt werden und die Differenz der Siedepunkte der Verbindungen (V) und (III) und die der Siedepunkte der Verbindungen (III) und (IV) jeweils mindestens 50°C beträgt, wobei der Katalysator A ein basischer Katalysator, ein Titan(IV)- oder Zirkonium(IV)-Alkanoat, ein Alkoxy-trialkyl-Zinn oder ein Kupferalkanoat/Triphenylphosphan-Komplex ist.

2. Verfahren gemäß Anspruch 1, **dadurch gekennzeichnet, dass** als Katalysator A Titan(IV)-oder Zirkonium(IV)-Alkanoat verwendet wird, bei dem das Alkanoat dem der Verbindung (III) entspricht.

3. Verfahren zur Herstellung der Verbindungen der Formel (I) 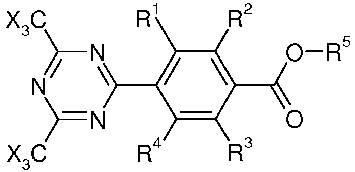 **dadurch gekennzeichnet, dass** X gleich Halogen und R¹, R², R³ und R⁴ unabhängig voneinander Wasserstoff, Halogen, Alkyl, Alkoxy, Alkenyl, Alkinyl, Alkylthio, Alkylseleno, wobei R¹ und R² und / oder R³ und R⁴ zusammen eine 3 bis 5-gliedrige gesättigte oder ungesättige gegebenenfalls mit bis zu 2 Heteroatomen substituierte Brücke sein können und R⁵ gegebenenfalls beliebig durch Halogen und / oder C₁ bis C₁₀-Alkyl-substituiertes und / oder C₁ bis C₁₀-Alkoxy-substituiertes lineares C₅ bis C₂₀-Alkyl, in dem bis zu 6 Kohlenstoff-Atome durch Sauerstoff ersetzt sein können, wobei zwischen zwei Sauerstoff-Atomen mindestens 2 Kohlenstoff-Atome vorhanden sein müssen und der Rest R⁵ mit mindestens 2 Kohlenstoff-Atomen beginnt, wobei die endständige Methyl-Gruppe des linearen C₅ bis C₂₀-Alkyl-Rests unsubstituiert sein muss, sind unter Verwendung des Verfahrens gemäß wenigstens einem der Ansprüche 1 oder 2, wobei in einem ersten Schritt das Ein-Topf-Verfahren zu Herstellung von para-disubstituierten Benzoesäurenitrilen der Formel (IV) angewendet wird und in einem zweiten Schritt das resultierende para-disubstituierten Benzoesäurenitril mit Trihalogenoacetonitril umgesetzt wird.

## Claims

1. Process for producing para-disubstituted benzonitriles of formula (IV) 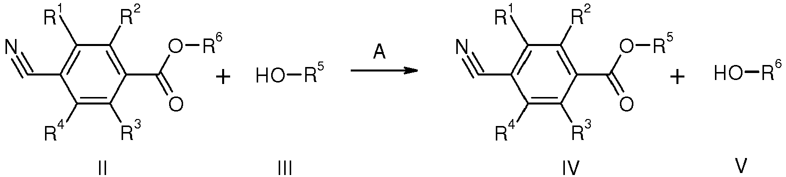 wherein R¹, R², R³ and R⁴ are each independently hydrogen, halogen, alkyl, alkoxy, alkenyl, alkynyl, alkylthio, alkylseleno, wherein R¹ and R² and/or R³ and R⁴ may combine to form a 3 to 5-membered saturated or unsaturated bridge optionally substituted with up to 2 heteroatoms and R⁵ is linear C₅ to C₂₀ alkyl which is optionally substituted in any manner by halogen and/or C₁ to C₁₀ alkyl and/or C₁ to C₁₀ alkoxy and in which up to six carbon atoms may be replaced by oxygen, in which case at least two carbon atoms shall be present between two oxygen atoms and the R⁵ moiety starts with at least two carbon atoms, wherein the terminal methyl group of the linear C₅ to C₂₀ alkyl moiety shall be unsubstituted and R⁶ is methyl, ethyl or isomeric propyl, and A is a catalyst A, in a one-pot process wherein component (III) is used in excess, components (V), (III) and (IV) are distillatively removed in the stated order in three steps by adjusting the pressure and the temperature, and the difference in the boiling points of compounds (V) and (III) and in the boiling points of compounds (III) and (IV) is not less than 50°C in either case, wherein said catalyst A is a basic catalyst, a titanium(IV) or zirconium(IV) alkanoate, an alkoxytrialkyltin or a copper alkanoate/triphenylphosphane complex.

2. Process according to Claim 1, **characterized in that** said catalyst A is a titanium (IV) or zirconium(IV) alkanoate wherein the alkanoate corresponds to that of compound (III).

3. Process for producing the compounds of formula (I) 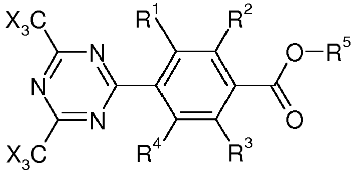 **characterized in that** X is halogen and R¹, R², R³ and R⁴ are each independently hydrogen, halogen, alkyl, alkoxy, alkenyl, alkynyl, alkylthio, alkylseleno, wherein R¹ and R² and/or R³ and R⁴ may combine to form a 3 to 5-membered saturated or unsaturated bridge optionally substituted with up to 2 heteroatoms and R⁵ is linear C₅ to C₂₀ alkyl which is optionally substituted in any manner by halogen and/or C₁ to C₁₀ alkyl and/or C₁ to C₁₀ alkoxy and in which up to six carbon atoms may be replaced by oxygen, in which case at least two carbon atoms shall be present between two oxygen atoms and the R⁵ moiety starts with at least two carbon atoms, wherein the terminal methyl group of the linear C₅ to C₂₀ alkyl moiety shall be unsubstituted by using the process according to at least one of Claims 1 or 2 which comprises utilizing the one-pot process to prepare para-disubstituted benzonitriles of formula (IV) in a first step and reacting the resulting para-disubstituted benzonitrile with trihaloacetonitrile in a second step.

## Revendications

1. Procédé de fabrication de nitriles de l'acide benzoïque para-disubstitués de la formule (IV) : 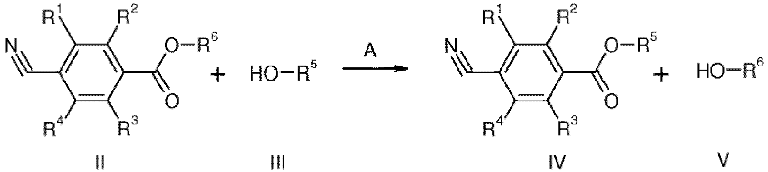 R¹, R², R³ et R⁴ représentant indépendamment les uns des autres l'hydrogène, un halogène, un alkyle, un alcoxy, un alcényle, un alcynyle, un alkylthio, un alkylséléno, R¹ et R² et/ou R³ et R⁴ pouvant représenter ensemble un pont de 3 à 5 éléments, saturé ou insaturé, éventuellement substitué avec jusqu'à 2 hétéroatomes, et R⁵ représentant un alkyle en C₅ à C₂₀ linéaire éventuellement substitué de manière quelconque par un halogène et/ou un alkyle en C₁ à C₁₀ et/ou substitué par un alcoxy en C₁ à C₁₀, dans lequel jusqu'à 6 atomes de carbone peuvent être remplacés par de l'oxygène, au moins 2 atomes de carbone devant être présents entre deux atomes d'oxygène, et le radical R⁵ débutant avec au moins 2 atomes de carbone, le groupe méthyle terminal du radical alkyle en C₅ à C₂₀ linéaire devant être non substitué, et R⁶ représentant méthyle, éthyle ou propyle isomère, et A représentant un catalyseur A, et le procédé consistant en un procédé monotope, selon lequel le composant (III) est utilisé en excès, les composants (V), (III) et (IV) sont séparés par distillation dans l'ordre indiqué en trois étapes par adaptation de la pression et de la température, et la différence entre les points d'ébullition des composés (V) et (III) et celle entre les points d'ébullition des composés (III) et (IV) est à chaque fois d'au moins 50 °C, le catalyseur A étant un catalyseur basique, un alcanoate de titane (IV) ou de zirconium (IV), un alcoxy-trialkyl-étain ou un complexe d'alcanoate de cuivre/triphénylphosphane.

2. Procédé selon la revendication 1, **caractérisé en ce qu'**un alcanoate de titane (IV) ou de zirconium (IV) est utilisé en tant que catalyseur A, l'alcanoate correspondant à celui du composé (III).

3. Procédé de fabrication des composés de la formule (I) : 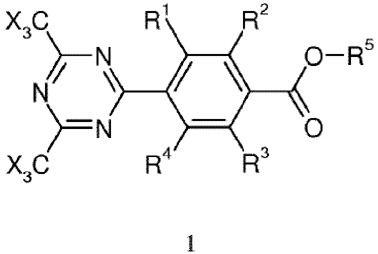 **caractérisé en ce que** X représente un halogène, et R¹, R², R³ et R⁴ représentent indépendamment les uns des autres l'hydrogène, un halogène, un alkyle, un alcoxy, un alcényle, un alcynyle, un alkylthio, un alkylséléno, R¹ et R² et/ou R³ et R⁴ pouvant représenter ensemble un pont de 3 à 5 éléments, saturé ou insaturé, éventuellement substitué avec jusqu'à 2 hétéroatomes, et R⁵ représente un alkyle en C₅ à C₂₀ linéaire éventuellement substitué de manière quelconque par un halogène et/ou un alkyle en C₁ à C₁₀ et/ou substitué par un alcoxy en C₁ à C₁₀, dans lequel jusqu'à 6 atomes de carbone peuvent être remplacés par de l'oxygène, au moins 2 atomes de carbone devant être présents entre deux atomes d'oxygène, et le radical R⁵ débutant avec au moins 2 atomes de carbone, le groupe méthyle terminal du radical alkyle en C₅ à C₂₀ linéaire devant être non substitué, en utilisant le procédé selon au moins l'une quelconque des revendications 1 ou 2, lors d'une première étape, le procédé monotope de fabrication de nitriles de l'acide benzoïque para-disubstitués de la formule (IV) étant appliqué et, lors d'une deuxième étape, le nitrile de l'acide benzoïque para-disubstitué résultant étant mis en réaction avec un trihalogénoacétonitrile.
